# EUROPÄISCHE PATENTSCHRIFT

(11) **EP 0 866 052 B1**
(45) Veröffentlichungstag und Bekanntmachung des Hinweises auf die Patenterteilung: **20.11.2002**
(21) Anmeldenummer: 98104139.5
(22) Anmeldetag: 09.03.1998
(51) Int. Cl.: C07C 209/62, C07C 209/68, C07C 211/36, C07C 209/86

(54) **Verfahren zur Herstellung von 1-Amino-1-methyl-3(4)-amino-methylcyclohexan**
Process for the preparation of 1-amino-1-methyl-3(4)-aminomethylcyclohexane
Procédé pour la préparation du amino-1-méthyl-3(4)-aminométhylcyclohexane

(30) Priorität: 20.03.1997 DE 19711548
(43) Veröffentlichungstag der Anmeldung: 23.09.1998
(73) Patentinhaber: BAYER AG, 51368 Leverkusen (DE)
(72) Erfinder: Fischer, Konrad, Dr., 51519 Odenthal (DE); Wilmes, Oswald, Dr., 51061 Köln (DE); Arlt, Dieter, Prof. Dr., 32657 Lemgo (DE); Casser, Carl, Dr., 51061 Köln (DE); Maas, Peter, 6365 Puth (DE); Woestenborghs, Pierre, 3650 Dilsen (BE); van der Knaap, Theo Dr., 6120 Grevenbicht (NL); Reintjens, Raf, 6360 Schimmert (NL)

(56) Entgegenhaltungen:
- EP-A- 0 153 561
- EP-A- 0 193 828
- EP-A- 0 415 213
- DE-A- 4 401 929

## Beschreibung

Die Erfindung betrifft ein neues Verfahren zur Herstellung von 1-Amino-1-methyl-3(4)-aminomethylcyclohexan (AMCA), dem Vorprodukt zur Herstellung von 1-Isocyanato-1-methyl-3(4)-isocyanatomethylcyclohexan (IMCI).

In der DE-A 4 401 929 wird zur Herstellung von 1-Amino-1-methyl-3(4)-aminomethylcyclohexan (AMCA) ein Verfahren beschrieben, das durch folgendes Reaktionsschema gekennzeichnet ist.

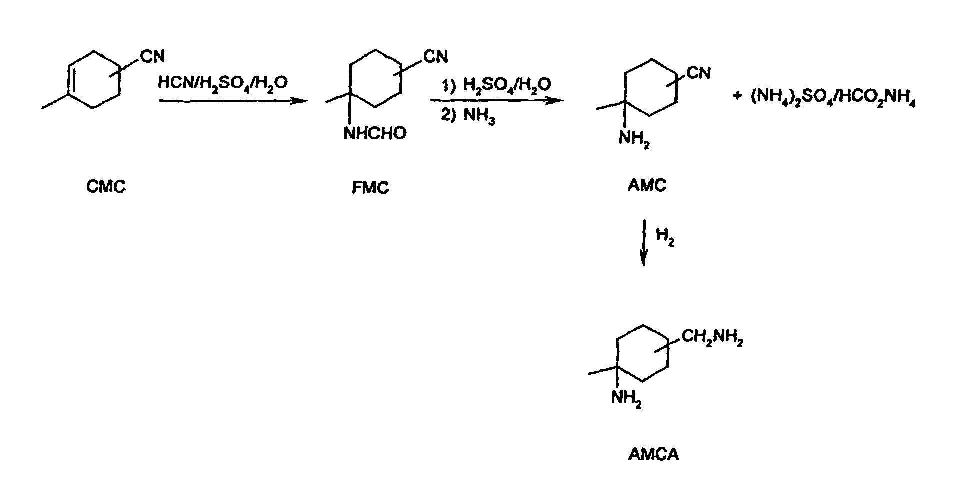

Nach diesem Vorschlag wird das als Ausgangsprodukt dienende 4(5)-Cyano-1-methylcyclohexen (CMC) mit Blausäure in Gegenwart von Schwefelsäure zum 1-Formamido-1-methyl-3(4)-cyanocyclohexan (FMC) umgesetzt, das nach destillativer Abtrennung überschüssiger Blausäure und Verdünnung des Reaktionsgemisches mit Wasser durch geeignete, mit Wasser nicht mischbare Lösungsmittel aus der sauren Wasserphase extrahiert werden kann. Eine andere, bekannte (siehe DE-A 4 401 929) Variante der Isolierung von FMC aus der nach der Ritterreaktion anfallenden wässrigen Reaktionsmischung besteht darin, diese saure Mischung ggf. nach Verdünnung mit Wasser mit Ammoniak zu neutralisieren, so dass das Gemisch einen Gehalt an Ammoniumsulfat von ca. 20 bis 70 Gew.-% aufweist und anschließend das im Gemisch vorliegende FMC mit Lösungsmitteln zu extrahieren. Obwohl bei dieser Variante der Isolierung von FMC aufgrund der Neutralisation der in der Ritterreaktion eingesetzten Schwefelsäure eine erhebliche Menge an Ammoniumsalze gebildet werden, handelt es sich hierbei um eine technisch relevante Verfahrensweise, da die Extraktion in diesem Falle auch mit geringen Extraktionsmittelmengen besonders vollständig erfolgt und dadurch die für die weitere Aufarbeitung aufzuwendende Energie verringert wird. In einem weiteren Reaktionsschritt wird anschließend das 1-Formamido-1-methyl-3(4)-cyanocyclohexan (FMC) im sauren, wässrigen Medium zum 1-Amino-1-methyl-3(4)-cyanocyclohexan (AMC) hydrolysiert.

Eine weitere aus der DE-A 4 401 929 bekannte Verfahrensvariante besteht darin, die Hydrolyse des 1-Formamido-1-methyl-3(4)-cyanocyclohexans (FMC) ohne vorherige Isolierung mit der im Ritterreaktionsgemisch vorliegenden Schwefelsäure durchzuführen.

Alle in der DE-A 4 401 929 beschriebenen Verfahrensvarianten sind dadurch gekennzeichnet, dass nach Hydrolyse des 1-Formamido-1-methyl-3(4)-cyanocylohexans eine saure, wässrige 1-Amino-1-methyl-3(4)-cyanocyclohexanlösung erhalten wird. Die im Verfahren sich anschließende extraktive Isolierung des 1-Amino-1-methyl-3(4)-cyanocyclohexans (AMC) kann nur aus alkalischer Lösung mit technisch ausreichendem Erfolg durchgeführt werden. Dies hat zur Konsequenz, dass zwangsläufig Salze als Nebenprodukte anfallen, die verwertet bzw. entsorgt werden müssen. Anschließend wird das so erhaltene 1-Amino-1-methyl-3(4)-cyanocyclohexan (AMC) ggf. nach destillativer Abtrennung des Extraktionsmittels in an sich bekannter Weise katalytisch zu 1-Amino-1-methyl-3(4)-aminomethylcyclohexan (AMCA) hydriert.

Die Alkalischstellung der nach Hydrolyse erhaltenen sauren, wässrigen 1-Amino-1-methyl-3(4)-cyanocyclohexanlösung wird in der Deutschen Patentanmeldung mit Ammoniak durchgeführt, so dass hierbei Ammoniumsulfat und Ammoniumformiat zwangsläufig als Nebenprodukte anfallen. In der o.g. Patentanmeldung wird vorgeschlagen dieses Ammoniumsulfat unter Abspaltung von zur Herstellung von Schwefelsäure wiederverwertbarem Schwefeldioxid einer thermolytischen Spaltung zu unterziehen. Aufgrund der hierfür notwendigen Aufkonzentration der wässrigen Ammoniumsulfatlösung ist diese Aufarbeitung der zwangsläufig anfallenden Nebenproduktsalze mit erheblichen Destillationskosten verbunden. Eine Aufarbeitung der nach AMC-Extraktion erhaltenen Salzlösung zu wiederverwertbarem Ammoniumsulfat ist ebenfalls mit erheblichen Kosten verbunden, da auch nach destillativer Entfernung des in der Salzlösung enthaltenen Extraktionsmittels und nach erfolgter Formiatentfernung die erhaltene Ammoniumsulfatlösung einen chemischen Sauerstoffbedarf (CSB) von 10g pro Liter aufweist.

Bei der in der DE-A 4 401 929 vorgesehenen katalytischen Hydrierung des 1-Amino-1-methyl-3(4)-cyanocyclohexans (AMC) zum 1-Amino-1-methyl-3(4)-aminomethylcyclohexan (AMCA) treten durch intramolekulare Cyclisierung und intermolekulare Dimerisierungen die Nebenprodukte I, II, III, IV, V 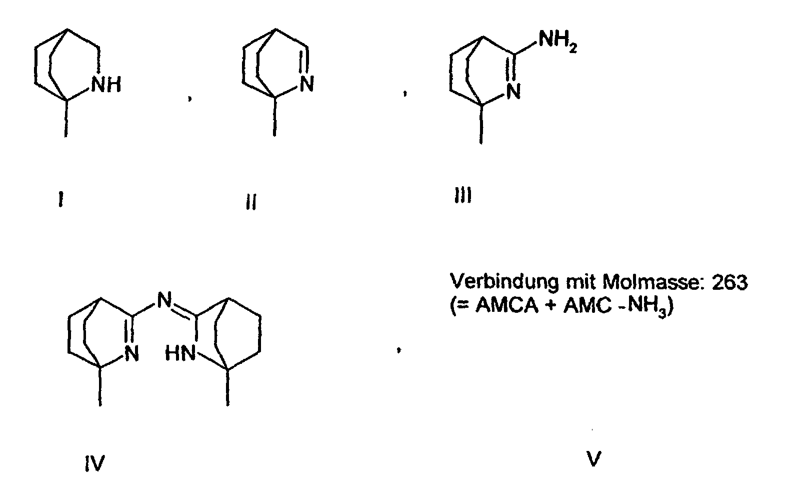 auf, so dass hierbei ein Selektivitätsverlust von ca. 20% entsteht.

Auch aus der EP-A 0 153 561 ist ein Verfahren zur Herstellung von AMCA bekannt, nach welchem ausgehend von 4(5)- Aminomethyl-1-methylcyclohexen durch Addition von Blausäure, anschließende alkalische Hydrolyse und abschließende Destillation die Zielverbindung neben einer zu entsorgenden Salzfracht und 4(3)-Aminomethyl-1-methylcycohexanol als Nebenprodukt erhalten werden kann.

Es wurde nun ein Verfahren zur Herstellung von 1-Amino-1-methyl-3(4)-aminomethylcyclohexan (AMCA) gefunden, welches eine deutlich höhere Gesamtselektivität (bezogen auf das Einsatzmaterial 4(5)-Cyano-1-methylcyclohexen (CMC)) aufweist und bei dem ggf. als Zwangsabfallprodukt ein ammoniumsulfathaltiges Abwasser entsteht, welches kostengünstig zu wiederverwertbarem Ammoniumsulfat aufgearbeitet werden kann.

Dieses Verfahren ist dadurch gekennzeichnet, dass man das nach dem in der DE-A 4 401 929 beschriebenen Verfahren erhaltene 1-Formamido-1-methyl-3(4)-cyanocyclohexan (FMC) aus der mit Wasser verdünnten und/oder mit Ammoniak neutralisierten Reaktionslösung durch Extraktion mit geeigneten Lösungsmitteln isoliert und anschließend ggf. nach destillativer Abtrennung des Extraktionsmittels katalytisch hydriert. Als Zwischenprodukte werden hierbei das 1-Formamido-1-methyl-3(4)-aminomethylcyclohexan (FMA) bzw. das hieraus durch intramolekulare Äquilibrierung erhaltene 1-Amino-1-methyl-3(4)-formamidomethylcyclohexan (AMF) bzw. durch intermolekulare Äquilibrierung das 1-Formamido-1-methyl-3(4)-formamidomethylcyclohexan (FMF) und AMCA erhalten.

Wird die Hydrierung in Gegenwart eines Formylierungsmittels durchgeführt, wird als neues Zwischenprodukt das 1-Formamido-1-methyl-3(4)-formamidomethylcyclohexan (FMF) erhalten. Anschließend werden die Zwischenprodukte, FMA, AMF, FMF mit alkalischen Verbindungen zu 1-Amino-1-methyl-3(4)-aminomethylcyclohexan (AMCA) und einem Ameisensäurederivat umgesetzt.

Das erfindungsgemäße Verfahren wird durch das folgende Formelschema beschrieben.

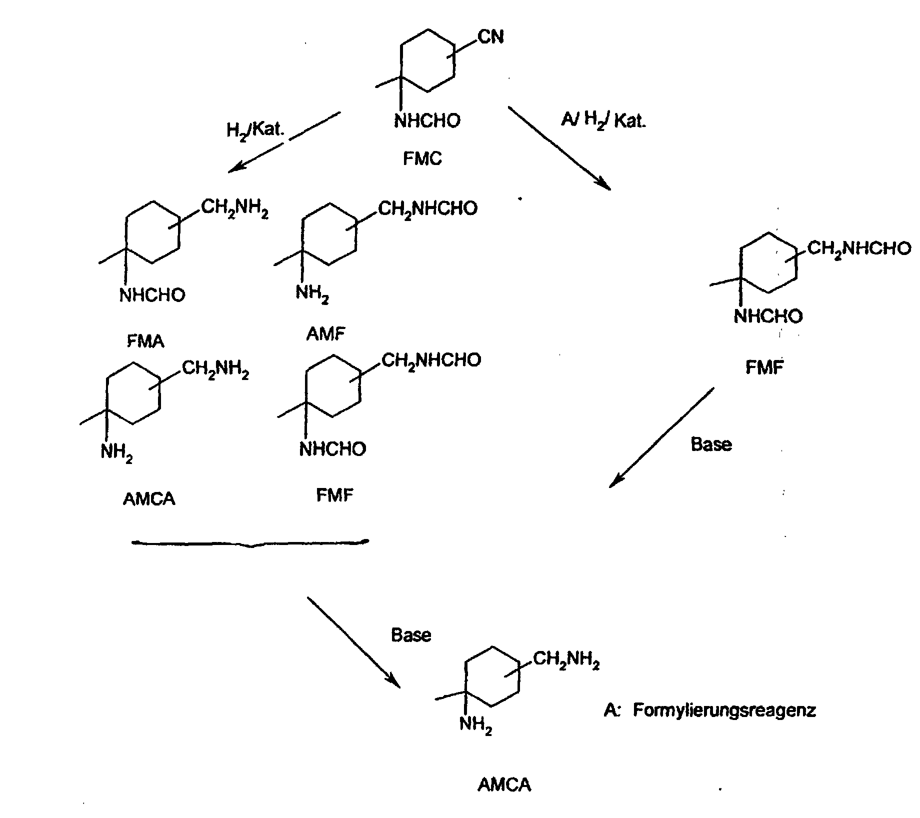

Die Reaktionsprodukte des erfindungsgemäßen Verfahrens (AMCA, Ameisensäurederivate) werden durch fraktionierte Destillation und/oder Kristallisätion und Filtration in reiner Form erhalten.

Gegenstand der Erfindung ist demzufolge ein Verfahren zur Herstellung von 1-Amino-1-methyl-3(4)-aminomethylcyclohexan (AMCA), ausgehend von 1-Formamido-1-methyl-3(4)-cyanocyclohexan (FMC), das in einer vorausgehenden Reaktionsstufe durch Umsetzung von 4(5)-Cyano-1-methylcyclohexen (CMC) mit Blausäure in Gegenwart von Schwefelsäure gebildet und nachfolgend aus der mit Wasser verdünnten oder mit Ammoniak neutralisierten Reaktionslösung durch Extraktion mit geeigneten Lösungsmitteln erhalten wurde, dadurch gekennzeichnet, dass man
a) in einer ersten Reaktionsstufe FMC katalytisch zu 1-Formamido-1-methyl-3(4)- aminomethylcyclohexan (FMA) und /oder 1-Amino-1-methyl-3(4)-form- amidomethylcyclohexan (AMF) und/oder 1-Formamido-1-methyl-3(4)-formamido-methylcyclohexan (FMF) und/oder AMCA hydriert und
b) in einer zweiten Reaktionsstufe FMA und /oder AMF und/oder FMF mit Ammoniak oder Alkali bzw. Erdalkalihydroxiden zu AMCA und Formamid bzw. zu AMCA und Alkali- oder Erdalkaliformiat umsetzt und
c) das erhaltene Reaktionsgemisch durch fraktionierte Destillation und/oder Kristallisation und Filtration in die Komponenten auftrennt.

Als Hydrierkatalysatoren werden Katalysatoren aus der Gruppe Ra-Ni, Ra-Co und/oder Ra-Fe in einer Menge von 1-20 Gew.-% bezogen auf FMC verwendet. Die Hydrierung wird bei Temperaturen von 50°C bis 200°C, bevorzugt bei Temperaturen von 60°C bis 160°C und unter einem Wasserstoffdruck von 50 bis 300 bar, bevorzugt unter 70 bis 200 bar durchgeführt.

Die Hydrierung kann auch in einem geeigneten Lösungsmittel, wie z.B. Hydroxyalkane, Alkylaromaten erfolgen, wobei das zur FMC-Extraktion dienende Extraktionsmittel bevorzugt ist. Die Hydrierung kann sowohl diskontinuierlich als auch kontinuierlich ausgeführt werden.

Die in der zweiten Reaktionsstufe erfolgende alkalische Spaltung von dem in der ersten Reaktionsstufe erhaltenen FMA und/oder AMF und/oder FMF mit Alkali- oder Erdalkalihydroxiden wird bei Temperaturen von 100°C bis 300°C, bevorzugt bei 120°C bis 250°C durchgeführt, wobei die Umsetzung auch in einem geeigneten Lösungsmittel, in dem das gebildete AMCA löslich und die gebildeten Alkali- oder Erdalkaliformiate unlöslich sind, durchgeführt werden kann. Geeignete Lösungsmittel sind z.B. Toluol und Diphenylether. Pro Mol FMA und/oder AMF werden ein bis zwei Mol Alkalihydroxid oder 0,5 bis ein Mol Erdalkalihydroxid verwendet. Bevorzugt wird ein Überschuss an Hydroxyläquivalenten von 2 bis 10 mol-% verwendet. Bevorzugte Alkali- und Erdalkalihydroxide sind Natriumhydroxid, Kaliumhydroxid und Calciumhydroxid.

Die in der zweiten Reaktionsstufe erfolgende alkalische Spaltung von dem in der ersten Stufe erhaltenen FMA und/oder AMF und/oder FMF mit Ammoniak wird bei Temperaturen von 100°C bis 300°C, bevorzugt bei 120°C bis 250°C durchgeführt, wobei die Umsetzung auch in einem geeigneten Lösungsmittel wie z.B. Hydroxyalkane durchgeführt werden kann.

Es ist auch möglich die erste und zweite Reaktionsstufe des erfindungsgemäßen Verfahrens in einer Eintopfreaktion durchzuführen. In diesem Falle wird die Hydrierung in Gegenwart von Ammoniak oder Alkali- und/oder Erdalkalihydroxiden ausgeführt, wobei nicht umgesetztes FMA und/oder AMF und/oder FMF nach Abtrennung von AMCA zurückgeführt werden.

Die Hydrierung des 1-Formamido-1-methyl-3(4)-cyanocyclohexans (FMC) kann auch in Gegenwart eines Formylierungsmittels erfolgen, wobei als neues Zwischenprodukt das Bisformamid, 1-Formamido-1-methyl-3(4)-formamidomethylcyclohexan (FMF), gebildet wird. Gegenstand der Erfindung ist demzufolge auch ein Verfahren zur Herstellung von 1-Amino-1-methyl-3(4)-aminomethylcyclohexan (AMCA), ausgehend von 1-Formamido-1-methyl-3(4)-cyanocyclohexan (FMC), das in einer vorausgehenden Reaktionsstufe durch Umsetzung von 4(5)-Cyano-1-methylcyclohexen (CMC) mit Blausäure in Gegenwart von Schwefelsäure gebildet und nachfolgend aus der mit Wasser verdünnten und/oder mit Ammoniak neutralisierten Reaktionslösung durch Extraktion mit geeigneten Lösungsmitteln erhalten wurde, dadurch gekennzeichnet, dass man
a) in einer ersten Reaktionsstufe FMC katalytisch in Gegenwart eines Formylierungsmittels zu 1-Formamido-1-methyl-3(4)-formamidomethylcyclohexan (FMF) hydriert und
b) in einer zweiten Reaktionsstufe das bei der Hydrierung erhaltene FMF mit Ammoniak oder Alkali- oder Erdalkalihydroxiden zu AMCA und Formamid bzw. zu AMCA und Alkali- oder Erdalkaliformiat umsetzt und
c) das erhaltene Reaktionsprodukt durch fraktionierte Destillation und/oder Kristallisation und Filtration in die Komponenten auftrennt.

Als Hydrierkatalysatoren werden Katalysatoren aus der Gruppe Ra-Ni, Ra-Co und/oder Ra-Fe in einer Menge von 1-20 Gew.-% bezogen auf FMC verwendet. Die Hydrierung wird bei Temperaturen von 50°C bis 200°C, bevorzugt bei Temperaturen von 60°C bis 160°C und unter einem Wasserstoffdruck von 50 bis 300 bar, bevorzugt unter 70-200 bar durchgeführt. Als Formylierungsmittel können Formamid, Alkylformiate und Kohlenmonoxid verwendet werden, wobei Methylformiat und Kohlenmonoxid bevorzugt sind. Die Hydrierung kann auch in einem geeigneten Lösungsmittel, wie z. B. Hydroxyalkane, Alkylaromaten erfolgen, wobei das zur FMC-Extraktion dienende Extraktionsmittel bevorzugt ist. Das Formylierungsmittel kann selbst als Lösungsmittel dienen. Die Hydrierung kann sowohl diskontinuierlich als auch kontinuierlich ausgeführt werden.

Die in der zweiten Reaktionsstufe erfolgende alkalische Spaltung des in der ersten Reaktionsstufe erhaltenen Bisformamids (FMF) wird bevorzugt mit Alkali- oder Erdalkalihydroxiden bei Temperaturen von 100°C bis 300°C, bevorzugt bei 120°C bis 250°C durchgeführt, wobei die Umsetzung auch in einem geeigneten Lösungsmittel, in dem das gebildete AMCA löslich und die gebildeten Alkali- oder Erdalkaliformiate unlöslich sind, ausgeführt werden kann. Geeignete Lösungsmittel sind z.B. Toluol und Diphenylether. Pro Mol FMF werden zwei bis drei Mol Alkalihydroxid oder ein bis 1,5 Mol Erdalkalihydroxid verwendet. Bevorzugt wird ein Überschuss an Hydroxyläquivalenten von 2 bis 10 mol-% verwendet. Bevorzugte Alkali- und Erdalkalihydroxide sind Natriumhydroxid, Kaliumhydroxid und Calciumhydroxid.

Die Isolierung des Ausgangsmaterials FMC aus dem Reaktionsgemisch der Ritterreaktion von CMC und Blausäure ist bekannt. Sie erfolgt durch Extraktion aus wässrig, saurem Reaktionsgemisch, dessen Schwefelsäureanteil nach Abdestillieren überschüssiger Blausäure ggf. durch Verdünnen mit Wasser auf ca. 10 - 70 %, vorzugsweise 10 - 50 %, eingestellt wurde.

In diesem Falle der Extraktion des Ausgangsmaterials FMC der vorliegenden Erfindung aus saurer Lösung ist es möglich, durch Aufkonzentration der zurückbleibenden wässrigen Phase die eingesetzte Schwefelsäure für die Ritterreaktion wiederzuverwenden.

Als Lösungsmittel, die zur Extraktion verwendet werden, eignen sich z.B. chlorierte Kohlenwasserstoffe wie Dichlormethan, Chloroform, Chlorbenzol, Ether wie z.B. t-Butylmethylether oder t-Butyloxy-2-propanol, Ketone wie z. B. Methylethylketon, Alkohole wie z.B. n- und i-Butanol, 1-Pentanol, 2-Methyl-1-butanol, 4-Methyl-2-pentanol und Cyclohexanol oder Gemische derartiger Lösungsmittel.

Nach Extraktion kann das Extraktionsmittel durch Destillation vom FMC abgetrennt und wiederverwendet werden. Das erhaltenen rohe FMC kann ggf. durch Destillation gereinigt werden; es ist jedoch bevorzugt, das rohe FMC der anschließenden Hydrierung direkt zuzuführen. In diesem Falle ist es möglich, die bei der FMC-Extraktion erhaltene Extraktphase ohne Abdestillation des Extraktionsmittels in der sich anschließenden Hydrierstufe einzusetzen, vorausgesetzt zur Extraktion wurden Lösungsmittel verwendet, die auch für die Hydrierung geeignet sind.

Eine andere, bekannte (siehe o.g. DE-A) Variante der Isolierung von FMC aus der nach der Ritterreaktion anfallenden wässrigen Reaktionsmischung besteht darin, diese saure Mischung ggf. nach Verdünnung mit Wasser mit Ammoniak zu neutralisieren, so dass das Gemisch einen Gehalt an Ammoniumsulfat von 20 bis 70 Gew.-% aufweist und anschließend das im Gemisch vorliegende FMC mit Lösungsmitteln zu extrahieren. Obwohl bei dieser Variante der Isolierung von FMC eine erhebliche Menge an Ammoniumsulfat gebildet wird, handelt es sich um eine technisch relevante Verfahrensweise, da die Extraktion in diesem Falle auch mit geringen Extraktionsmittelmengen besonders vollständig erfolgt und dadurch die für die weitere Aufarbeitung aufzuwendenden Energien verringert werden. Das bei dieser Variante der FMC-Isolierung erhaltene ammoniumsulfathaltige Abwasser zeichnet sich durch einen geringen CSB-Wert aus, so dass hieraus kostengünstig Ammoniumsulfat als Wertprodukt isoliert werden kann.

Das nach den verschiedenen Varianten des erfindungsgemäßen Verfahrens erhaltene AMCA kann in an sich bekannter Weise zu IMCI phosgeniert werden. In den nachfolgenden Beispielen beziehen sich alle Prozentangaben auf das Gewicht.

### Beispiele

### Beispiel 1

Zu einer Mischung aus 5,4 Mol 88%iger Schwefelsäure und 891 g Blausäure werden bei 27 bis 29°C innerhalb von 75 min unter Rühren 363 g 4(5)-Cyano-1-methylcyclohexen (Isomerengemisch, erhalten durch Cycloaddition von Isopren und Acrylnitril) zudosiert, wobei die Reaktionswärme durch Rückflusskühlung der siedenden Blausäure abgeführt wird. Zehn Minuten nach Beendigung der Eduktzugabe werden 900g Wasser zugegeben und die überschüssige Blausäure abdestilliert. Dann werden bei 20 bis 30°C 735 g 25%ige wässrige Ammoniaklösung zudosiert. Die erhaltene neutralisierte Reaktionslösung wird anschließend fünfmal mit je 150 g i-Butanol extrahiert. Man erhält nach Entfernen des Extraktionsmittels 525 g rohes 1-Formamido-1-methyl-3(4)-cyanocyclohexan (Isomerenmischung) mit einem gaschromatographisch ermitteltem Gehalt von 88,5%. Das in der nach Extraktion erhaltenen wässrige Ammoniumsulfatlösung gelöste i-Butanol wird destillativ entfernt. Die verbleibende, vom i-Butanol befreite wässrige Ammoniumsulfatlösung (1800 g, Schwefelgehalt: 9,5% entspricht 39,5% Ammoniumsulfat) weist einen chemischen Sauerstoffbedarf (CSB) von 3500 mg/l auf.

### Vergleichsbeispiel 1

175 g rohes 1-Formamido-1-methyl-3(4)-cyanocyclohexan aus Beispiel 1 werden in 476 g 37%iger Schwefelsäure gelöst und 6 Stunden auf 60°C erwärmt. Anschließend wird durch Zugabe von 25%iger Ammoniaklösung der pH-Wert der Reaktionslösung auf 1,8 eingestellt und die in der Reaktionslösung enthaltene Ameisensäure durch Extraktion mit i-Butanol entfernt. Die nach Ameisensäureextraktion erhaltene wässrige Phase wird durch Zugabe von 25%iger Ammoniaklösung auf einen pH-Wert von 10 eingestellt und sechsmal mit je 300g i-Butanol extrahiert. Nach Entfernen des i-Butanols werden 157 g rohes 1-Amino-1-methyl-3(4)-cyanoylcyclohexan (Isomerenmischung) mit einem gaschromatographisch ermitteltem Gehalt von 80,9 % erhalten. Die verbleibende vom i-Butanol und Ammoniak befreite wässrige Ammoniumsulfatlösung wird auf einen Schwefelgehalt von 9,5 % eingestellt und weist einen chemischen Sauerstoffbedarf (CSB) von 10500 mg/l auf.

Das Vergleichsbeispiel zeigt, dass bei der Isolierung von 1-Amino-1-methyl-3(4)-cyanocyclohexan (AMC) im Vergleich zur Isolierung von 1-Formamido-1-methyl-3(4)-cyanocyclohexan (FMC) sich ein ammoniumsulfathaltiges Abwasser mit deutlich höherem CSB-Wert ergibt, so dass im Falle der AMC-Isolierung bei der weiteren Aufarbeitung dieses Abwassers zu wiederverwertbarem Ammoniumsulfat deutlich höhere Kosten anfallen.

### Beispiel 2

175 g rohes 1-Formamido-1-methyl-3(4)-cyanocyclohexan aus Beispiel 1 werden in 400g Methylformiat gelöst und unter Zugabe von 9 g Ra-Ni bei einem Wasserstoffdruck von 150 bar und einer Temperatur von 150°C hydriert. Nach beendeter Wasserstoffaufnahme wird der Katalysator filtriert und überschüssiges Methylformiat und gebildetes Methanol abdestilliert. Es verbleiben 210 g rohes 1-Formamido-1-methyl-3(4)-formamidomethylcyclohexan mit einem gaschromatographisch ermitteltem Gehalt von 83,3 %.

### Vergleichsbeispiel 2

157 g rohes 1-Amino-1-methyl-3(4)-cyanocyclohexan aus Vergleichsbeispiel 1 werden in Gegenwart von 200 g Methanol, 300 ml Ammoniak und 9 g Ra-Ni bei einem Wasserstoffdruck von 100 bar und einer Temperatur von 100 bis 120°C hydriert. Nach beendeter Wasserstoffaufnahme wird der Katalysator filtriert und Methanol und Ammoniak destillativ entfernt. Es verbleiben 146 g rohes 1-Amino-1-methyl-3(4)-aminomethylcyclohexan mit einem gaschromatographisch ermitteltem Gehalt von 73,4 %. Ca. 20 Gew.-% entfallen auf die Summe der Verbindungen der Formeln I, II, III, IV und V.

### Beispiel 3

210 g rohes 1-Formamido-1-methyl-3(4)-fonnamidomethylcyclohexan aus Beispiel 2 werden in Gegenwart von 300 g Toluol und 75 g Natriumhydroxid sechs Stunden auf 180°C erhitzt. Nach Filtration des gebildeten Natriumformiats und Destillation des Toluols verbleibt ein Rückstand von 143 g an roh 1-Amino-1-methyl-3(4)-aminomethylcyclohexan mit einem gaschromatographisch ermitteltem Gehalt von 85,9 %.

Aus den Beispielen 1 bis 3 des erfindungsgemäßen Verfahrens ergibt sich eine Gesamtselektivität (bezogen auf das Einsatzmaterial CMC) von 86,5 %. Für das über das 1-Amino-1-methyl-3(4)-cyanocyclohexan (AMC) verlaufende Verfahren ergibt sich hingegen nur eine Gesamtselektivität von 75,5 % (siehe Vergleichsbeispiele 1 und 2).

### Beispiel 4

175g rohes 1-Formamido-1-methyl-3(4)-cyanocyclohexan aus Beispiel 1 werden in 1,3 1 Methanol und 0,9 1 Ammoniak unter Zusatz von 9 g Ra-Ni bei einem Wasserstoffdmck von 100 bar und einer Temperatur von 140°C bis zur Beendigung der Wasserstoffaufnahme hydriert. Nach Filtration des Katalysators und Destillation von Methanol und Ammoniak verbleiben 180 g Rückstand mit einem gaschromatographisch ermittelten Gehalt von 30 % 1-Amino-1-methyl-3(4)-aminomethylcyclohexan, 40 % 1-Formamido-1-methyl-3(4)-aminomethylcyclohexan mit 1-Amino-1-methyl-3(4)-formamidomethylcyclohexan und 13 % 1-Formamido-1-methyl-3(4)-formamidomethylcyclohexan.

## Patentansprüche

1. Verfahren zur Herstellung von 1-Amino-1-methyl-3(4)-aminomethylcyclohexan (AMCA), ausgehend von 1-Formamido-1-methyl-3(4)-cyanocyclohexan (FMC), das in einer vorausgehenden Reaktionsstufe durch Umsetzung von 4(5)-Cyano-1-methylcyclohexen (CMC) mit Blausäure in Gegenwart von Schwefelsäure gebildet und nachfolgend aus der mit Wasser verdünnten und/oder mit Ammoniak neutralisierten Reaktionslösung durch Extraktion erhalten wurde, **dadurch gekennzeichnet, dass** man
a) in einer ersten Reaktionsstufe FMC katalytisch zu 1-Formamido-1-methyl-3(4)-aminomethylcyclohexan (FMA) und/oder 1-Amino-1-methyl-3(4)-formamidomethylcyclohexan (AMF) und/oder 1-Formamido-1-methyl-3(4)-formamidomethylcyclohexan (FMF) und/oder AMCA hydriert und
b) in einer zweiten Reaktionsstufe FMA und/oder AMF und/oder FMF mit Ammoniak oder Alkali bzw. Erdalkalihydroxiden zu AMCA und Formamid bzw. zu AMCA und Alkali- oder Erdalkaliformiat umsetzt und
c) das erhaltene Reaktionsgemisch durch fraktionierte Destillation und/oder Kristallisation und Filtration in die Komponenten auftrennt.

2. Verfahren gemäß Anspruch 1, **dadurch gekennzeichnet, dass** man als Hydrierkatalysator einen Katalysator aus der Gruppe Ra-Ni, Ra-Co und/oder Ra-Fe verwendet.

3. Verfahren gemäß Anspruch 1, **dadurch gekennzeichnet, dass** man die Hydrierung in einem Lösungsmittel durchführt.

4. Verfahren gemäß Anspruch 1, **dadurch gekennzeichnet, dass** man in der zweiten Reaktionsstufe Ammoniak als alkalische Verbindung verwendet und das hierbei erhaltene AMCA und Formamid durch fraktionierte Destillation auftrennt und ggf. nicht umgesetztes FMA und/oder AMF und/oder FMF in die Reaktionsstufe zurückführt.

5. Verfahren gemäß Anspruch 1, **dadurch gekennzeichnet, dass** man in der zweiten Reaktionsstufe Alkali- und/oder Erdalkalihydroxide als alkalische Verbindung verwendet und die hierbei erhaltenen Formiatsalze und AMCA durch Kristallisation, Filtration und Destillation auftrennt und ggf. nicht umgesetztes FMA und/oder AMF und/oder FMF in die Reaktionsstufe zurückführt.

6. Verfahren gemäß Anspruch 5, **dadurch gekennzeichnet, dass** man die zweite Reaktionsstufe in einem Lösungsmittel durchführt, in dem das gebildete AMCA löslich und die gebildeten Formiatsalze unlöslich sind.

7. Verfahren gemäß Anspruch 1, **dadurch gekennzeichnet, dass** man die Hydrierung von FMC in Gegenwart eines Formylierungsmittels durchführt und das hierbei erhaltene 1-Formamido-1-methyl-3(4)-formamidomethylcyclohexan (FMF) in der zweiten Reaktionsstufe mit einer alkalischen Verbindung zu AMCA und zu einem Derivat der Ameisensäure umsetzt und das erhaltene Reaktionsgemisch durch fraktionierte Destillation und/oder Kristallisation und Filtration in die Komponenten auftrennt.

8. Verfahren gemäß Anspruch 7, **dadurch gekennzeichnet, dass** man als Formylierungsmittel Formamid und/oder Ameisensäureester und/oder Kohlenmonoxid verwendet.

9. Verfahren gemäß Anspruch 1, **dadurch gekennzeichnet, dass** man die Hydrierung von FMC und die alkalische Spaltung des gebildeten FMA und/oder AMF und/oder FMF in einer Eintopfreaktion ausführt.

## Claims

1. A method of preparing 1-amino-1-methyl-3(4)-aminomethylcyclohexane (AMCA) from 1-formamido-1-methyl-3(4)-cyanocyclohexane (FMC) which was formed in a preceding reaction step by the reaction of 4(5)-cyano-1-methylcyclohexene (CMC) with hydrocyanic acid in the presence of sulphuric acid and which was subsequently obtained by extraction from the reaction solution which was diluted with water and/or neutralised with ammonia, **characterised in that**
a) in a first reaction step, FMC is catalytically hydrogenated to form 1-formamido-1-methyl-3(4)-aminomethylcyclohexane (FMA) and/or 1-amino-1-methyl-3(4)-formamidomethylcyclohexane (AMF) and/or 1-formamido-1-methyl-3(4)-formamidomethylcyclohexane (FMF) and/or AMCA, and
b) in a second reaction step, FMA and /or AMF and/or FMF are reacted with ammonia or with alkali or alkaline earth metal hydroxides to form AMCA and formamide or to form AMCA and an alkali or alkaline earth metal formate, respectively, and
c) the reaction mixture which is obtained is separated into its components by fractional distillation and/or by crystallisation and filtration.

2. A method according to claim 1, **characterised in that** a catalyst from the group comprising Raney Ni, Raney Co and/or Raney Fe is used as a hydrogenation catalyst.

3. A method according to claim 1, **characterised in that** hydrogenation is conducted in a solvent.

4. A method according to claim 1, **characterised in that** in the second reaction step ammonia is used as an alkaline compound and the AMCA and formamide which are thereby obtained are separated by fractional distillation and any unreacted FMA and/or AMF and/or FMF are recycled to the reaction step.

5. A method according to claim 1, **characterised in that** in the second reaction step alkali and/or alkaline earth metal hydroxides are used as an alkaline compound and the formate salts which are thereby obtained are separated by crystallisation, filtration and distillation, and any unreacted FMA and/or AMF and/or FMF are recycled to the reaction step.

6. A method according to claim 5, **characterised in that** the second reaction step is conducted in a solvent in which the AMCA which is formed is soluble and in which the formate salts which are formed are insoluble.

7. A method according to claim 1, **characterised in that** the hydrogenation of FMC is conducted in the presence of a formylation agent and the 1-formamido-1-methyl-3(4)-formamidomethyl-cyclohexane (FMF) which is thereby obtained is reacted in the second reaction step with an alkaline compound to form AMCA and a derivative of formic acid, and the reaction mixture obtained is separated into its components by fractional distillation and/or by crystallisation and filtration.

8. A method according to claim 7, **characterised in that** formamide and/or esters of formic acid and/or carbon monoxide are used as formylation agents.

9. A method according to claim 1, **characterised in that** the hydrogenation of FMC and the alkaline cleavage of the FMA and/or AMF and/or FMF which are formed are carried out as a one-pot reaction.

## Revendications

1. Procédé pour la préparation du 1-amino-1-méthyl-3(4)-aminométhylcyclohexane (AMCA) à partir du 1-formamido-1-méthyl-3(4)-cyanocyclohexane (FMC) qui a été formé dans une étape réactionnelle préalable par mise en réaction du 4(5)-cyano-1-méthylcyclohexène (CMC) avec de l'acide cyanhydrique en présence d'acide sulfurique et que l'on a ensuite obtenu par extraction à partir de la solution réactionnelle diluée avec de l'eau et/ou neutralisée avec de l'ammoniac, **caractérisé en ce que**
a) dans une première étape réactionnelle, on soumet du FMC à une hydrogénation catalytique pour obtenir du 1-formamido-1-méthyl-3(4)-aminométhylcyclohexane (FMA) et/ou du 1-amino-1-méthyl-3(4)-formamidométhylcyclohexane (AMF) et/ou du 1-formamido-1-méthyl-3(4)-formamidométhylcyclohexane (FMF) et/ou du AMCA et
b) dans une deuxième étape réactionnelle, on fait réagir du FMA et/ou du AMF et/ou du FMF avec de l'ammoniac ou avec des hydroxydes de métaux alcalins, respectivement des hydroxydes de métaux alcalino-terreux pour obtenir du AMCA et du formamide respectivement pour obtenir du AMCA et du formiate de métal alcalin ou de métal alcalino-terreux, et
c) on sépare en ses composants le mélange réactionnel obtenu, par distillation fractionnée et/ou par cristallisation et par filtration.

2. Procédé selon la revendication 1, **caractérisé en ce qu'**on utilise, à titre de catalyseur de l'hydrogénation, un catalyseur choisi parmi le groupe comprenant du nickel de Raney, du cobalt de Raney et/ou du fer de Raney.

3. Procédé selon la revendication 1, **caractérisé en ce qu'**on effectue l'hydrogénation dans un solvant.

4. Procédé selon la revendication 1, **caractérisé en ce qu'**on utilise, dans la deuxième étape, de l'ammoniac à titre de composé alcalin et on sépare le AMCA et le formamide que l'on obtient en l'occurrence, par distillation fractionnée et, le cas échéant, on renvoie dans l'étape réactionnelle le FMA et/ou le AMF et/ou le FMF n'ayant pas réagi.

5. Procédé selon la revendication 1, **caractérisé en ce qu'**on utilise, dans la deuxième étape réactionnelle, des hydroxydes de métaux alcalins et/ou des hydroxydes de métaux alcalino-terreux, à titre de composé alcalin et on sépare les sels de formiates et le AMCA que l'on obtient en l'occurrence, par cristallisation, par filtration et par distillation et, le cas échéant, on renvoie dans l'étape réactionnelle le FMA et/ou le AMF et/ou le FMF n'ayant pas réagi.

6. Procédé selon la revendication 5, **caractérisé en ce qu'**on effectue la deuxième étape réactionnelle dans un solvant dans lequel est soluble le AMCA qui s'est formé et dans lequel sont insolubles les sels de formiates qui se sont formés.

7. Procédé selon la revendication 1, **caractérisé en ce qu'**on effectue l'hydrogénation du FMC en présence d'un agent de formylation et on fait réagir le 1-formamido-1-méthyl-3(4)-formamidométhylcyclohexane (FMF) que l'on obtient en l'occurrence, dans la deuxième étape réactionnelle, avec un composé alcalin pour obtenir du AMCA et un dérivé de l'acide formique et on sépare le mélange réactionnel obtenu en ses composants, par distillation fractionnée et/ou par cristallisation et par filtration.

8. Procédé selon la revendication 7, **caractérisé en ce qu'**on utilise à titre d'agent de formylation, le formamide et/ou l'ester de l'acide formique et/ou le monoxyde de carbone.

9. Procédé selon la revendication 1, **caractérisé en ce qu'**on effectue l'hydrogénation du FMC et le clivage alcalin du FMA et/ou du AMF et/ou du FMF qui se sont formés, dans une réaction en un seul pot.
